# EUROPÄISCHE PATENTSCHRIFT

(11) **EP 3 006 081 B1**
(45) Veröffentlichungstag und Bekanntmachung des Hinweises auf die Patenterteilung: **15.05.2019**
(21) Anmeldenummer: 15185274.6
(22) Anmeldetag: 15.09.2015
(51) Int. Cl.: A61N 1/362, A61N 1/39, H01M 2/10

(54) **EXTERNER HERZSCHRITTMACHER/KARDIOVERTER**
EXTERNAL PACEMAKER/CARDIOVERTER
CARDIOVERTEUR/STIMULATEUR CARDIAQUE EXTERNE

(30) Priorität: 09.10.2014 DE 102014014999
(43) Veröffentlichungstag der Anmeldung: 13.04.2016
(73) Patentinhaber: Osypka AG, 79618 Rheinfelden-Herten (DE)
(72) Erfinder: Kerl, Ferdinand, 91054 Erlangen (DE); Madzar, Goran, 90768 Fürth (DE); Bosch, Martin, 90765 Fürth (DE); Grämmer, Norbert, 90449 Nürnberg (DE); Tröger, Tobias, 91052 Erlangen (DE)
(74) Vertreter: Herrmann, Uwe

(56) Entgegenhaltungen:
- DE-A1-102009 001 611
- US-A- 5 413 499
- US-A- 5 637 417

## Beschreibung

Die vorliegende Erfindung betrifft einen externen Herzschrittmacher und/oder externen Kardioverter mit wenigstens einem Gehäuse, wobei das Gehäuse wenigstens ein Batteriefach mit einem oder mehreren Batteriefachkontakten aufweist und wobei das Batteriefach wenigstens eine Schublade mit einem Aufnahmebereich zur Aufnahme von zumindest einer Batterie umfasst.

Bei aus dem Stand der Technik bekannten externen Herzschrittmachern ist das Batteriefach in Form einer Schublade ausgebildet, die in das Gehäuse eingeschoben und aus diesem herausgezogen werden kann.

Des Weiteren sind externe Herzschrittmacher bekannt, die ein einfaches Batteriefach aufweisen, in das die Batterie eingelegt wird.

Eine Berührung der Batteriefachkontakte oder der mit den Batteriefachkontakten in elektrischer Verbindung stehenden Batterie kann bei kombinierten Herzschrittmachern / Kardiovertern aufgrund der anliegenden Spannungen (z.B. 400V) für den Anwender ein erhebliches Risiko einer gesundheitlichen Gefährdung durch einen elektrischen Schlag mit sich bringen.

Abgesehen davon muss eine Berührung der Batteriefachkontakte bzw. der mit diesen verbundenen Batterie durch das medizinische Personal verhindert werden, da ansonsten unter Umständen (z. B. durch kurze ESD-Pulse) ungewollte Ströme über die Herzdrähte und das Herz des Patienten fließen können und dadurch die Herzaktivität beeinflusst werden aknn (zu hohe Patientenableitströme). Dies ist darauf zurück zu führen, dass zwischen dem medizinischen Personal und dem Herzen des Patienten eine direkte elektrische Verbindung über die Batterie bzw. die Batteriefachkontakte, die interne Schaltung des Gerätes, die Gerätebuchsen und die metallischen Herzdrähte bestehen kann.

Der vorliegenden Erfindung liegt daher die Aufgabe zugrunde, einen externen Herzschrittmacher und/oder einen externen Kardioverter, vorzugsweise einen externen Herzschrittmacher mit kombinierten Kardioverter dahingehend weiterzubilden, dass die Wahrscheinlichkeit für das Auftreten einer Gefährdung für den Anwender bzw. Patienten minimiert wird.

Diese Aufgabe wird durch einen Herzschrittmacher und/oder Kardioverter mit den Merkmalen des Anspruchs 1 gelöst. Danach ist vorgesehen, dass der oder/die Batteriefachkontakte nicht oder nicht alle in oder an der Schublade angeordnet sind.

Dadurch lässt sich der Vorteil erreichen, dass bei ausgezogener Schublade kein Batteriefachkontakt oder jedenfalls nicht alle Batteriefachkontakte zugänglich sind, sodass eine versehentliche Berührung nicht stattfinden kann. Ein weiterer Vorteil besteht darin, dass bei in den Aufnahmebereich eingelegter Batterie und geöffneter Schublade die Batterie selbst mit keinem Batteriefachkontakt oder jedenfalls nicht mit allen Batteriefachkontakten in Verbindung steht, sodass deren Berührung durch den Anwender keine nachteiligen Folgen hat.

Vorzugsweise ist vorgesehen, dass zumindest eine bzw. mehrere oder alle Batteriefachkontakte ortsfest in dem Gehäuse angeordnet sind. Vorzugsweise ist vorgesehen, dass kein Batteriefachkontakt in oder an der Schublade angeordnet ist.

Die Batterie kann somit gefahrlos in den Aufnahmebereich der Schublade eingesetzt werden. Eine elektrische Verbindung des Gerätes mit der Batterie findet somit erst dann statt, wenn die Schublade zumindest teilweise und vorzugsweise vollständig eingeschoben ist. Im vollständig ausgezogenen Zustand der Schublade, in dem die Batterie eingesetzt wird, ist vorgesehen, dass die Batterie nicht oder jedenfalls nicht vollständig mit den Batteriefachkontakten verbunden ist.

Somit können durch die Maßnahmen gemäß der Erfindung die Erfordernisse der Norm IEC 60601-1 erfüllt werden.

Vorzugsweise ist des Weiteren vorgesehen, dass zumindest eine oder alle Batteriefachkontakte an der Position des Gehäuses angeordnet sind, die die Schublade bzw. die eingelegte Batterie mit ihrem in Einschubrichtung vorlaufenden Ende nach zumindest dem halben Einschubweg und besonders bevorzugt im vollständig eingeschobenen Zustand der Schublade erreicht.

Eine besonders vorteilhafte Ausgestaltung besteht darin, wenn zumindest einer und vorzugsweise alle Batteriefachkontakte derart angeordnet sind, dass die eingelegte Batterie bzw. die Schublade mit ihrem in Einschubrichtung vorlaufenden Ende diese erst im vollständig eingeschobenen Zustand erreicht. Darunter ist zu verstehen, dass die Batterie erst dann mit dem bzw. mit allen Batteriefachkontakten in elektrisch leitender Verbindung steht, wenn die Schublade vollständig eingeschoben ist.

In einer weiteren bevorzugten Ausgestaltung der Erfindung ist vorgesehen, dass zumindest einer oder alle Batteriefachkontakte durch wenigstens eine Wandung des Gehäuses überdeckt sind, wenn die Schublade geöffnet ist, sodass eine Berührung der Batteriefachkontakte bei geöffneter Schublade durch einen Anwender verhindert wird. Bei der fraglichen Wandung, die eine solche unmittelbare Berührung verhindert bzw. erschwert kann es sich beispielsweise um die Rückwand des Gehäuses handeln. Grundsätzlich sind auch andere Wandungen des Gehäuses geeignet, eine solche unmittelbare Berührung zu verhindern oder zumindest zu erschweren, wie beispielsweise die Seitenwände etc.

In einer weiteren Ausgestaltung der Erfindung ist vorgesehen, dass der Aufnahmebereich der Schublade zur Gehäuseunterseite hin offen ist. Zum Einsetzen der Batterie wird somit das Gerät gewendet, sodass dessen Vorderseite unten liegt und sodann die Schublade geöffnet. Die Batterie kann dann von oben oder z.B. auch von der Seite in die Schublade eingeführt werden.

Bevorzugt ist es, wenn der Aufnahmebereich der Schublade im eingeschobenen Zustand der Schublade vollständig von Wandungen umgeben ist. Damit ist sichergestellt, dass ein Berühren der Batteriefachkontakte und/oder der Batterie im vollständig eingeschobenen Zustand der Schublade durch den Anwender ausgeschlossen ist.

In einer bevorzugten Ausgestaltung der Erfindung ist vorgesehen, dass die Schublade gegen Herausfallen gesichert ist. Somit kann zumindest ein Anschlag oder dergleichen vorgesehen sein, der verhindert, dass die Schublade über eine bestimmte Position hinaus ausgezogen werden kann.

Grundsätzlich ist ein Verriegelungsmechanismus vorgesehen, der mit der Schublade derart zusammenwirkt, dass diese nur nach Betätigung des Verriegelungsmechanismus, d.h. nach einer Entriegelung geöffnet werden kann.

Die Verriegelung ist durch einen Schieber verdeckt, der zunächst durch den Anwender verschoben werden muss, bevor die Verriegelung frei liegt und somit eine Entriegelung erfolgen kann. Denkbar ist es, dass der genannte Schieber durch eine Feder in eine Ausgangsposition positioniert wird, in der der Schieber die Verriegelung der Schublade verdeckt.

Denkbar ist es, dass der Schieber zum Freilegen der Verriegelung gegen die Federkraft einer Feder bewegt werden muss. Dies hat den Vorteil, dass im nicht betätigten Zustand der Schieber die Verriegelung abdeckt und somit keine versehentliche Entriegelung stattfinden kann.

US5,637,417 beschreibt einen externen Herzschrittmacher, der eine Batterieaufnahme-Schublade mit Verriegelung umfasst. Zu der Verriegelung und dem Schieber wird Bezug genommen auf die DE 102013008314, die eine solche Ausführungsform offenbart.

Weitere Einzelheiten und Vorteile der Erfindung werden anhand eines in der Zeichnung dargestellten Ausführungsbeispiels näher erläutert. Es zeigen:
- Figur 1:: Eine Außenansicht eines kombinierten Herzschrittmachers und Kardioverters mit geschlossener Batterieschublade mit Ansicht auf die Geräteunterseite,
- Figur 2:: Eine Außenansicht gemäß Figur 1 mit geöffneter Batterieschublade,
- Figur 3:: Eine Außenansicht gemäß Figur 2 beim Einlegen der Batterie,
- Figur 4:: Eine Außenansicht gemäß Figur 3 mit vollständig eingelegter Batterie und
- Figur 5:: Eine Innenansicht des kombinierten Herzschrittmachers/Kardioverters mit geschlossener Batterieschublade in einer Ansicht von der Geräteoberseite mit geöffneter Oberschale des Gehäuses.

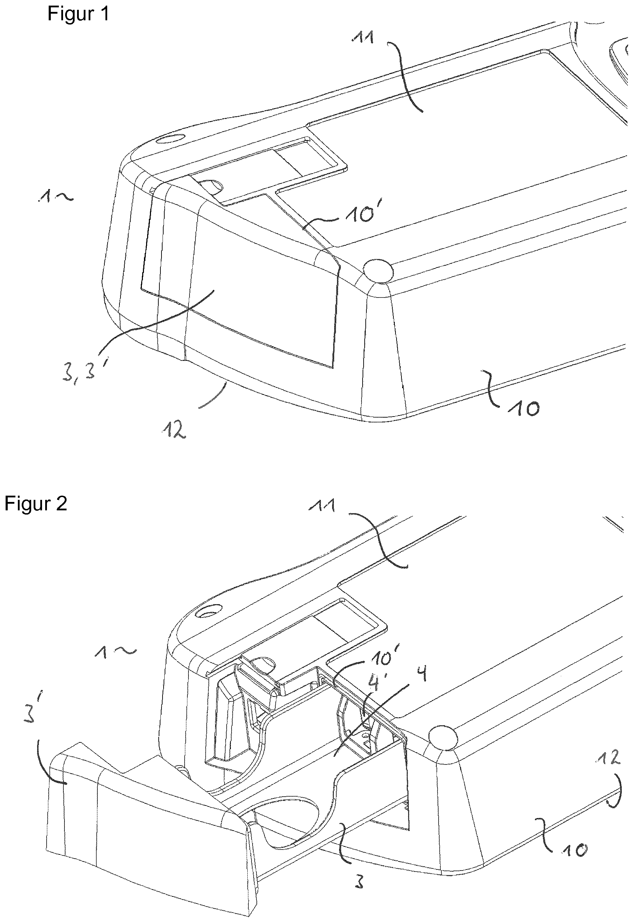

Figur 1 zeigt einen kombinierten externen Herzschrittmacher mit Kardioverter, der ein Gehäuse 1 aufweist, in dem sich die für den Betrieb des Gerätes erforderlichen elektrischen Schaltungen befinden.

Das Gehäuse 1 weist umlaufende Seitenwandungen 10, eine Rückwandung 11 und eine frontseitige Wandung 12 auf. Das Gehäuse 1 ist somit insgesamt geschlossen.

Eine der Seitenwandungen sowie ein Teilbereich der Rückwand 11 weist einen ausgesparten Bereich 10' auf, der durch die Front 3' einer Schublade 3 geschlossen ist, wie dies aus Figur 1 hervorgeht.

Außer der Front 3' weist die Schublade einen Aufnahmebereich 4 auf, der zur Aufnahme einer Batterie 5, beispielsweise zur Aufnahme einer 9V-Batterie dient. Der Aufnahmebereich 4 ist durch eine Bodenfläche, die Front 3' sowie zwei seitliche Wandungen begrenzt. Wie dies aus Figur 2 hervorgeht, ist der Aufnahmebereich 4 nach oben hin offen, wenn das Gerät mit der nach oben gewandten Rückwand 11 angeordnet ist.

Das Bezugszeichen 4' kennzeichnet die in Einschubrichtung vorlaufende Wandung der Schublade 3.

Keine der Wandungen der Schublade 3 ist mit Batteriefachkontakten ausgeführt.

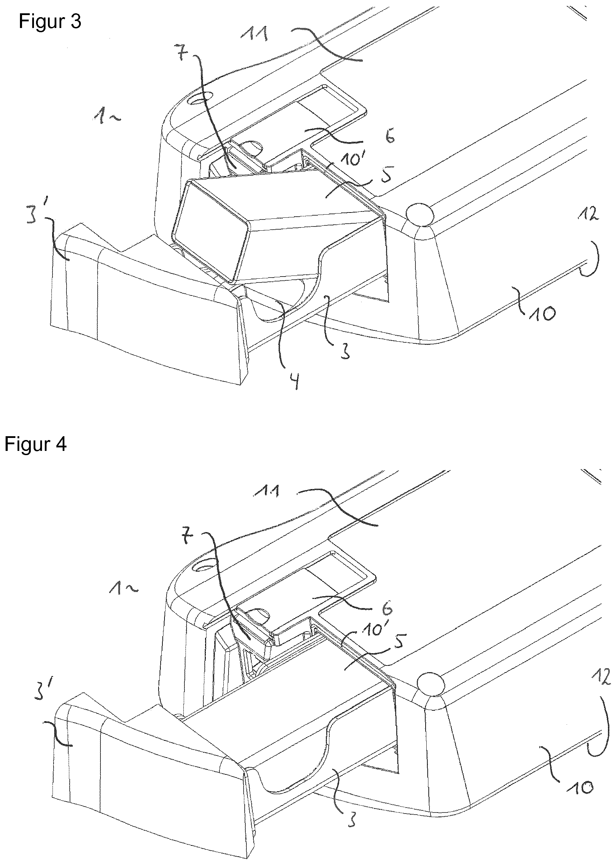

Figur 3 zeigt den Vorgang des Einlegens der Batterie in den Aufnahmebereich 4 der Schublade 3 und Figur 4 zeigt den Zustand, in dem die Batterie 5 vollständig in den Aufnahmebereich 4 der Schublade 3 angeordnet ist. Wie dies aus den Figuren hervorgeht, wird die Batterie 5 so eingelegt, dass ihre stirnseitigen Kontakte in Einschubrichtung weisen.

Weder während des Einlegens gemäß Figur 3 noch im eingelegten Zustand gemäß Figur 4 steht die Batterie 5 mit Batteriefachkontakten in Verbindung.

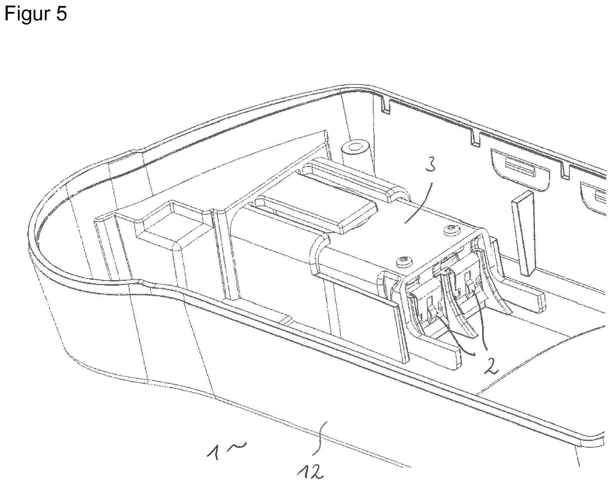

Die ortsfest angeordneten Batteriefachkontakte sind in Figur 5 mit dem Bezugszeichen 2 gekennzeichnet.

Wie dies aus Figur 5 hervorgeht, befinden sich im Gehäuse 1 zwei nebeneinander angeordnete Batteriefachkontakte 2, die im vollständig eingeschobenen Zustand der Schublade 3 mit den beiden vorlaufend angeordneten Kontakten der hier exemplarisch dargestellten 9V-Batterie in Verbindung stehen.

Die Batteriefachkontakte 2 sind ortsfest angeordnet und werden nicht mit der Schublade 3 bewegt.

Die Batteriefachkontakte 2 sind so am Gehäuse angeordnet, dass diese erst dann mit den Gegenkontakten der Batterie 5 in Kontakt stehen, wenn die Schublade 3 vollständig eingeschoben ist. Dieser Zustand ist in Figur 1 und 5 dargestellt.

Wie oben ausgeführt, kann der Anwender nach dem Öffnen der Schublade 3 diese bis zum Anschlagspunkt aus dem Gerät herausziehen, bis die in Figur 2 dargestellte Position erreicht ist. Eine Berührung der Batteriefachkontakte im Gerät durch den Anwender wird durch die Rückwand der Schublade 3, d.h. durch die in Einschubrichtung vorlaufende Wand der Schublade 3 verhindert, da die Batteriefachkontakte 2 nicht in der Schublade 3, sondern am Ende des Einschubweges der Schublade, d.h. am Ende des Weges des eingeschobenen Batteriefachs montiert sind.

Nachdem der Anwender die Batterie 5 in die Schublade 3 eingelegt hat, wie dies aus den Figuren 3 und 4 hervorgeht, berührt diese die im Batteriefach montierten Batteriefachkontakte 2 nicht, solange die Batterieschublade 3 nicht geschlossen ist. Somit kann die Batterie 5 gefahrlos berührt werden, da keine elektrische Verbindung mit der internen Schaltung des Geräts besteht.

Das Batteriefach umfasst somit die bewegbar angeordnete Schublade 3 sowie die ortsfest angeordneten Kontakte 2.

Durch das Schließen und ggf. durch das Verriegeln der Batterieschublade 3 wird die Batterie 5 mit den im Gehäuse 1 montierten Batteriekontakten 2 und somit mit der internen Schaltung des Gerätes elektrisch verbunden.

Wie dies aus Figur 1 hervorgeht, befindet sich im Bereich neben der Schublade 3 ein Schieber 6, der einen unterhalb des Schiebers angeordneten Verriegelungsmechanismus 7 bedeckt. Der Schieber 6 wird durch eine Feder in der Figur 1 dargestellten Position gehalten.

Soll die Verriegelung 7 betätigt werden und somit ein Öffnen der Schublade 3 ermöglicht werden, muss der Anwender bzw. Patient den Schieber 6 gemäß Figur 1 nach rechts verschieben, sodass der darunter liegende Verriegelungsmechanismus 7 frei liegt. Nach Betätigung der Verriegelung 7 kann die Schublade 3 dann händisch oder durch Federkraft rausgeschoben werden.

Denkbar ist es, dass eine elektrische Verbindung zwischen der Batterie 5 und den Batteriefachkontakten 2 erst dann eintritt, wenn die Schublade 3 vollständig geschlossen ist und zusätzlich verriegelt ist. Denkbar ist es auch, dass eine solche Kontaktierung bereits dann stattfindet, wenn die Batterieschublade 3 vollständig in das Gerät eingeschoben ist, unabhängig davon, ob eine Verriegelung existiert bzw. ob eine Verriegelungsposition vorliegt.

## Patentansprüche

1. Externer Herzschrittmacher und/oder Kardioverter mit wenigstens einem Gehäuse (1), wobei das Gehäuse (1) wenigstens ein Batteriefach mit einem oder mehreren Batteriefachkontakten (2) aufweist und wobei das Batteriefach wenigstens eine Schublade (3) mit einem Aufnahmebereich (4) zur Aufnahme von zumindest einer Batterie (5) umfasst, wobei der oder die Batteriefachkontakte (2) nicht oder nicht alle in oder an der Schublade (3) angeordnet sind, **dadurch gekennzeichnet, dass** die Schublade (3) mittels einer von einem Schieber (6) verdeckbaren Verriegelung gesichert ist

2. Externer Herzschrittmacher und/oder Kardioverter nach Anspruch 1, **dadurch gekennzeichnet, dass** wenigstens einer oder alle Batteriefachkontakte (2) ortsfest in dem Gehäuse (1) angeordnet sind und/oder dass kein Batteriefachkontakt (2) in oder an der Schublade (3) angeordnet ist.

3. Externer Herzschrittmacher und/oder Kardioverter nach Anspruch 1 oder 2, **dadurch gekennzeichnet, dass** wenigstens einer oder alle Batteriefachkontakte (2) an der Position des Gehäuses (1) angeordnet sind, die eine in der Schublade (3) befindliche Batterie (5) mit ihrem in Einschubrichtung vorlaufenden Ende im vollständig eingeschobenen Zustand der Schublade (3) erreicht.

4. Externer Herzschrittmacher und/oder Kardioverter nach einem der vorhergehenden Ansprüche, **dadurch gekennzeichnet, dass** wenigstens einer oder alle Batteriefachkontakte (2) auch bei geöffneter Schublade (3) durch wenigstens eine Wandung des Gehäuses (1) überdeckt sind, so dass die Berührung durch einen Anwender verhindert wird.

5. Externer Herzschrittmacher und/oder Kardioverter nach Anspruch 4, **dadurch gekennzeichnet, dass** es sich bei der Wandung des Gehäuses (1) ausschließlich oder auch um die Rückwand (11) des Gehäuses (1) handelt.

6. Externer Herzschrittmacher und/oder Kardioverter nach einem der vorhergehenden Ansprüche, **dadurch gekennzeichnet, dass** zwei oder mehr als zwei Batteriefachkontakte (2) vorgesehen sind, die vorzugsweise nebeneinander oder übereinander angeordnet sind.

7. Externer Herzschrittmacher und/oder Kardioverter nach einem der vorhergehenden Ansprüche, **dadurch gekennzeichnet, dass** der Aufnahmebereich (4) der Schublade (3) bei geöffneter Schublade (3) zur Gehäuseunterseite hin offen ist.

8. Externer Herzschrittmacher und/oder Kardioverter nach einem der vorhergehenden Ansprüche, **dadurch gekennzeichnet, dass** der Aufnahmebereich (4) der Schublade (3) im eingeschobenen Zustand der Schublade (3) vollständig von Wandungen umgeben ist.

9. Externer Herzschrittmacher und/oder Kardioverter nach Anspruch 8, **dadurch gekennzeichnet, dass** der Schieber (6) durch eine Feder in einer Ausgangsposition positionierbar ist, in der der Schieber (6) die Verriegelung der Schublade (3) verdeckt, wobei vorzugsweise vorgesehen ist, dass der Schieber (6) zum Freilegen der Verriegelung (7) gegen die Federkraft der Feder bewegbar ist.

10. Externer Herzschrittmacher und/oder Kardioverter nach einem der vorhergehenden Ansprüche, **dadurch gekennzeichnet, dass** Mittel vorgesehen sind, die die Schublade (3) gegen ein Herausfallen sichern.

## Claims

1. An external pacemaker and/or cardioverter having at least one housing (1), wherein the housing (1) has at least one battery compartment having one or more battery compartment contacts (2); and wherein the battery compartment comprises at least one drawer (3) having a reception region (4) for receiving at least one battery (5), wherein the battery compartment contact (2) or the battery compartment contacts (2) is not or are not all arranged in or at the drawer (3), **characterized in that** the drawer (3) is secured by means of a latch coverable by a slider (6).

2. An external pacemaker and/or cardioverter in accordance with claim 1, **characterized in that** at least one battery compartment contact (2) or all the battery compartment contacts (2) is/are arranged in a fixed position in the housing (1); and/or **in that** no battery compartment contact (2) is arranged in or at the drawer (3).

3. An external pacemaker and/or cardioverter in accordance with claim 1 or claim 2, **characterized in that** at least one battery compartment contact (2) or all the battery compartment contacts (2) is/are arranged at the position of the housing (1) that the end of a battery (5) located in the drawer (3) leading in the push-in direction reaches in the completely pushed-in state of the drawer (3).

4. An external pacemaker and/or cardioverter in accordance with one of the preceding claims, **characterized in that** at least one battery compartment contact (2) or all the battery compartment contacts (2) is/are also covered by at least one wall of the housing (1) with an open drawer (3) so that a contact by a user is prevented.

5. An external pacemaker and/or cardioverter in accordance with claim 4, **characterized in that** the wall of the housing (1) is only or also the rear wall (11) of the housing (1).

6. An external pacemaker and/or cardioverter in accordance with one of the preceding claims, **characterized in that** two or more than two battery compartment contacts (2) are provided that are preferably arranged next to one another or above one another.

7. An external pacemaker and/or cardioverter in accordance with one of the preceding claims, **characterized in that** the reception region (4) of the drawer (3) is open toward the lower housing side with an open drawer (3).

8. An external pacemaker and/or cardioverter in accordance with one of the preceding claims, **characterized in that** the reception region (4) of the drawer (3) is completely surrounded by walls in the pushed-in state of the drawer (3).

9. An external pacemaker and/or cardioverter in accordance with claim 8, **characterized in that** the slider (6) is positionable by a spring in a starting position in which the slider (6) covers the latch of the drawer (3), with provision preferably being made that the slider (6) is movable against the spring force of the spring to expose the latch (7).

10. An external pacemaker and/or cardioverter in accordance with one of the preceding claims, **characterized in that** means are provided that secure the drawer (3) against falling out.

## Revendications

1. Stimulateur cardiaque externe et/ou cardioverteur comprenant au moins un boîtier (1), le boîtier (1) comportant au moins un compartiment à piles doté d'un ou de plusieurs contacts de compartiment à piles (2) et le compartiment à piles comprenant au moins un tiroir (3) doté d'une zone de réception (4) pour recevoir au moins une pile (5), le ou les contacts de compartiment à piles (2) n'étant pas ou pas tous disposés dans ou sur le tiroir (3), **caractérisé en ce que** le tiroir (3) est fixé au moyen d'un mécanisme de verrouillage pouvant être caché par un élément coulissant (6).

2. Stimulateur cardiaque externe et/ou cardioverteur selon la revendication 1, **caractérisé en ce qu'**au moins un ou tous les contacts de compartiment à piles (2) sont disposés fixement dans le boîtier (1) et/ou **en ce qu'**aucun contact de compartiment à piles (2) n'est disposé dans ou sur le tiroir (3).

3. Stimulateur cardiaque externe et/ou cardioverteur selon la revendication 1 ou 2, **caractérisé en ce qu'**au moins un ou tous les contacts de compartiment à piles (2) sont disposés sur l'emplacement du boîtier (1) qu'une pile (5) se trouvant dans le tiroir (3) atteint avec son extrémité avant dans le sens d'insertion lorsque le tiroir (3) est complètement rentré.

4. Stimulateur cardiaque externe et/ou cardioverteur selon l'une des revendications précédentes, **caractérisé en ce qu'**au moins un ou tous les contacts de compartiment à piles (2) sont recouverts par au moins une paroi du boîtier (1) lorsque le tiroir (3) est ouvert, de sorte qu'un utilisateur ne peut pas le ou les toucher.

5. Stimulateur cardiaque externe et/ou cardioverteur selon la revendication 4, **caractérisé en ce que** la paroi du boîtier (1) est exclusivement ou également la paroi arrière (11) du boîtier (1).

6. Stimulateur cardiaque externe et/ou cardioverteur selon l'une des revendications précédentes, **caractérisé en ce que** deux contacts de compartiment à piles (2) ou plus sont prévus, qui sont disposés de préférence les uns à côté des autres ou au-dessus des autres.

7. Stimulateur cardiaque externe et/ou cardioverteur selon l'une des revendications précédentes, **caractérisé en ce que** la zone de réception (4) du tiroir (3) est ouverte vers la face inférieure du boîtier lorsque le tiroir (3) est ouvert.

8. Stimulateur cardiaque externe et/ou cardioverteur selon l'une des revendications précédentes, **caractérisé en ce que** la zone de réception (4) du tiroir (3) est complètement entourée de parois lorsque le tiroir (3) est rentré.

9. Stimulateur cardiaque externe et/ou cardioverteur selon la revendication 8, **caractérisé en ce que** l'élément coulissant (6) peut être positionné par un ressort dans une position initiale, dans laquelle l'élément coulissant (6) cache le mécanisme de verrouillage du tiroir (3), l'élément coulissant (6) étant de préférence prévu mobile contre la force élastique du ressort pour découvrir le mécanisme de verrouillage (7).

10. Stimulateur cardiaque externe et/ou cardioverteur selon l'une des revendications précédentes, **caractérisé en ce que** des moyens qui empêchent le tiroir (3) de tomber sont prévus.
